# EUROPEAN PATENT APPLICATION

(11) **EP 1 845 376 A1**
(43) Date of publication of application: **17.10.2007**
(21) Application number: 06712408.1
(22) Date of filing: 26.01.2006
(51) Int. Cl.: G01N 33/542, C12N 15/09, G01N 21/64, C12N 15/00, C07K 16/18

(54) **QUANTITATIVE MEASUREMENT METHOD FOR RECOMBINANT PROTEIN**

(30) Priority: 28.01.2005 JP 2005020856
(71) Applicant: Shionogi Co., Ltd., Osaka-shi, Osaka 541-0045 (JP)
(72) Inventor: UWABE, Ken-ichiro, onaka-shi, Osaka, 5610825 (JP); ENOMOTO, Koji, hima-ku, Osaka-shi, Osaka, 5530002 (JP); ONODA, Junji, onaka-shi, Osaka, 5610825 (JP); NUMATA, Yoshito, hima-ku, Osaka-shi, Osaka, 5530002 (JP); TAKEMOTO, Hiroshi, hima-ku, Osaka-shi, Osaka, 5530002 (JP)
(74) Representative: Gillard, Marie-Louise
(86) International application number: PCT/JP2006/301226
(87) International publication number: WO 2006/080396

(57) **Abstract**

The present invention is intended to provide a method of rapidly, simply and accurately measuring a recombinant protein. As means for resolution, the protein quantity is measured by causing the expression of a fusion protein with the target protein and epitope tags containing two types of epitopes, bringing them into contact with detection antibodies which specifically recognize each epitope, and detecting a phenomenon caused by both detection antibodies coming close to each other.

## Description

### Technological Field

The present invention concerns a rapid quantitative measurement method for recombinant proteins, using epitope tags containing two types of epitopes.

### Prior Art

In the study of proteins, represented by "proteomes", one of the most important techniques is that of introducing a cloned gene into the cells, causing a recombinant protein to be expressed, conducting separation and refinement, and then studying the qualities and functions of the protein. Moreover, proteins are also very important in the screening of new drugs and in drug manufacturing, and the expression, separation and refinement of recombinant proteins are unavoidable steps. When causing a recombinant protein to be expressed, separating and refining it, the quantity expressed is sometimes smaller than expected, and sometimes the protein expressed forms an insoluble precipitate. It is therefore necessary to determine the optimal conditions for the expression, separation and refinement, and while exploring these conditions, there are often opportunities to measure the quantity of the recombinant protein.
In such a case, generally, after refining by using a tag, SDS-polyacryl-amide gel electrophoresis, ELISA, western blot and other immunoassays (non-patent document 1) are used. Many tags are known to improve the refining efficiency in such a process (non-patent document 2).
Non-patent document 1: "Practical Medicine Supplementary Volume: Handbook for Protein Tests", pp 128 to 134, Yodosha, 2004
Non-patent document 2: K. Terpe, Appl Microbiol Biotechnol, pp 523 to 533, 2003

### Disclosure of the Invention

### Problems Which the Invention Is Intended to Solve

However, in the existing quantitative measurement methods of recombinant proteins, because processes aimed at separating the protein from other materials, such as washing, are necessary, complex operation processes and time are required. Furthermore, the existing quantitative measurement methods of recombinant proteins are only capable of qualitatively confirming the expression of recombinant protein, and are incapable of accurately measuring its quantity. Moreover, accurately measuring the quantity of the target recombinant protein when the culture medium contains a mixture has been problematic.
In light of the above, the objective of the present invention is to provide a quantitative method of rapidly, simply and accurately measuring a recombinant protein.

### Means Used to Solve the Above Problem(s)

In other words, the present invention proposes,
[1] a method for quantitative measurement of target proteins, which is characterized in that it consists of a process in which, in a fusion protein of an epitope tag, containing epitope 1 and epitope 2, and a target protein, the detection antibody that recognizes epitope 1 and the detection antibody that recognizes epitope 2 are brought into contact, and the phenomenon produced by bringing close together said two detection antibodies is detected, and in that said epitope 1 and epitope 2, in the case that the detection antibodies that recognize each of them are bonded, are placed so that these two detection antibodies can be brought close together,
[2] the method according to [1] above, whereby said detection antibodies are labeled by means of a fluorescent material, illuminant, radioisotope or beads,
[3] the method according to [2] above, whereby said detection antibodies are labeled by a fluorescent material,
[4] the method according to [3] above, whereby said fluorescent material is a combination of a europium compound and an allophycocyanin derivative,
[5] the method according to any of [1] through [4] above, whereby said phenomenon is energy transfer by fluorescent resonance,
[6] the method according to [1] above, whereby in one of said detection antibodies, the amino acid sequence of the VH region is the one shown in sequence no. 4 and the amino acid sequence of the VL region is the one shown in sequence no. 5,
[7] the method according to [1] or [6] above, whereby in one of said detection antibodies, the amino acid sequence of the VH region is the one shown in sequence no. 6 and the amino acid sequence of the VL region is the one shown in sequence no. 7,
[8] the method according to [1] above, whereby each of said epitopes consists of 6 to 8 amino acid residues,
[9] the method according to [8] above, whereby the amino acid sequence of one of said epitopes is the one shown in sequence no. 1,
[10] the method according to [8] or [9] above, whereby the amino acid sequence of one of said epitopes is the one shown in sequence no. 2,
[11] the method according to [1] above, whereby epitope 1 and epitope 2 are mutually linked via a peptide linker,
[12] the method according to [11] above, whereby said peptide linker consists of 3 to 6 amino acid residues,
[13] the method according to [1] above, whereby said epitope tag consists of 15 to 22 amino acid residues,
[14] the method according to [13] above, whereby the amino acid sequence of said epitope tag is the one shown in sequence no. 3,
[15] an expression vector containing a gene consisting of the sequence shown in sequence no. 14,
[16] a kit for quantitative measurement of target proteins, containing detection antibodies that recognize epitope 1, detection antibodies that recognize epitope 2, and an expression vector that contains a gene consisting of a gene sequence that codes the epitope tag that consists of epitope 1 and epitope 2,
[17] and the kit for quantitative measurement of target proteins according to [16] above, containing the detection antibodies according to [6] above, the detection antibodies according to [7] above, and the expression vector according to [15] above.
[18] A kit which uses the method according to Claim [1].
[19] A detection antibody that recognizes epitope 1 and a detection antibody that recognizes epitope 2, and a fusion protein complex of an epitope tag, consisting of epitope 1 and epitope 2, and the target protein.

### Effect of the Invention

By implementing the present invention, quantitative measurement of recombinant protein, which required considerable time with the existing techniques, can be done within a short time. Moreover, since it enables quantitative measurement without requiring purification or other separation processes, it makes possible simple and accurate quantitative measurement of recombinant proteins without any complex steps. It is possible, therefore, to measure the quantity of a recombinant protein even when the culture medium, in which the recombinant protein is expressed, contains other proteins. In addition, the present invention can be widely used on various types of protein.

### A Brief Description of the Figures

[Fig. 1] A figure showing an example of an expression vector of the epitope tag fusion protein pertaining to the present invention.
[Fig. 2] A figure showing the measurement results of the epitope tag fusion protein, obtained by means of western blot using 6G4 antibody and 2E6 antibody.
[Fig. 3] A graph showing the results of studying the effect of the extract of animal cells, *Escherichia coli,* fission yeast and insect cells and medium components on measurements on the amount of epitope tag fusion protein, pertaining to the present invention, using epitope tag fusion SPDS as a reference material.
[Fig. 4] A graph showing the results of studying the relationships between the coefficients of variation of the measured value and the quantity of reference material on measurements of the epitope tag fusion protein, pertaining to the present invention, using epitope tag fusion SPDS as a reference material.
[Fig. 5] A table showing the results obtained when actually measuring the epitope tag fusion protein pertaining to the present invention for a transient expression system of HEK293 cells.

### Best Configuration for Implementing the Invention

The present invention is a method of measuring the quantity of a target protein, which is characterized in that it consists of a process in which, in the fusion protein of the epitope tag, containing epitope 1 and epitope 2, and the target protein, the detection antibody that recognizes epitope 1 and the detection antibody that recognizes epitope 2 are brought into contact, and the phenomenon produced by bringing close together said two detection antibodies is detected, and in that said epitope 1 and epitope 2, in the case that the detection antibodies that recognize each of them are bonded, are placed so that these two detection antibodies can be brought close together. The invention will now be described in detail.

The present invention is characterized in that it contains the following processes (1) through (3).
(1) An epitope tag, containing epitope 1 and epitope 2, is fused to a target protein and a fusion protein is produced. Then, if the following detection antibodies that recognize each of epitope 1 and epitope 2 are bonded, a process that places both said detection antibodies so that the can come close together.
(2) A process whereby the detection antibody that recognizes epitope 1 and the detection antibody that recognizes epitope 2 are brought into contact with said fusion protein.
(3) A process whereby a phenomenon, produced by the detection antibodies, bonded to both epitopes, coming close to each other, is detected. As the target protein, used in the implementation of the present invention, the DNA coding said protein can be determined, and of course the protein expressed by the DNA can be produced and used. Furthermore, naturally-occurring proteins, their mutants, artificial proteins and their mutants can also be used. As naturally-occurring proteins, proteins transcribed or translated from the cDNA library, which are derived from organs, tissues or cells of various animals, can be used. As artificial proteins, sequences obtained by combining all or some amino acid sequences of naturally-occurring proteins, or proteins containing a random amino acid sequence, can be used, among others. In the following implementation examples, a method of measuring the quantity of spermidine synthetase (SPDS) as a target protein is used.

Antibody-bonding regions on an antigen molecule are called epitopes. While no special restrictions are placed on the type of antibody and of the epitope of the antibody, in the implementation of the present invention, the antibody and the epitope of the antibody must be specified. Moreover, epitopes capable of linking to a target protein are called epitope tags; using an antigen-antibody reaction, they can be used for tracking, separation and refinement of the protein with which the tag is fused. In the present invention, the epitope tag is characterized in that it contains at least two types of epitopes (for example, epitope 1 and epitope 2).

The amino acid sequences of the epitopes, used in the implementation of the present invention, must be specified. In specifying the epitopes sequences, known amino acid sequences can be used or new sequences can be designed for each epitope. In this process, the epitope should be constructed of 6 to 10 amino acid residues, preferably 6 to 8 amino acid residues. For example, FLAG (DYKDDDDK, Sigma, registered trademark), c-myc (EQKLISEEL), polyhistidine (HHHHHH) and the like are epitopes commonly known to those skilled in the art, and since antibodies that can specifically bond with these epitopes are commercially sold, purchasing the antibodies is also easy. The epitope can be used as the epitope pertaining to the present invention as long as these amino acid sequences are included, and any amino acids can then be added before or after them. Moreover, in the event that a new amino acid sequence is designed, if the epitope's ability to specifically bond with the antibody is confirmed, using the methods described below, it can be used as the epitope pertaining to the present invention.

Methods of specifying the epitope of an antibody include (A) a method of determination by specifying the amino acid sequence that specifically bonds with the antibody, and (B) a method whereby an antibody is produced by immunization by a specific peptide antigen. The method of implementing the present invention is not limited, however, to or by any of these methods.
(A) An example of a method of determining the amino acid sequence that specifically bonds with an antibody, is the method of specifying it by means of western blot (Proc. Natl. Acad. Sci. U.S.A. 76, 3116 (1979)), using said antibody. Specifically, if a protein was used as the antigen, first the DNA that codes the protein is split by restriction enzymes into fragments that code 50-200 amino acid units, each of the DNA fragments is inserted into an appropriate expression vector, transcribed and translated by a suitable host, and a protein is expressed, and the bondability of the protein with the antibody is checked. A suitable expression vector can be any vector that suits the host into which it is inserted. Examples of the host include *Escherichia coli,* yeast, animal cells, insect cells etc., and pET, pNMT, pcDNA and pFastBac (all of which can be purchased from Invitrogen), respectively, can be used. Moreover, a non-cellular transcription-translation system, prepared based on rabbit reticulocytes, extracted wheat germ, *Escheriehia coli* extract *(Escherichia coli* S30 extract) or the like, can also be used as the system for transcription and translation. By means of the above, the epitope is focused to a peptide fragment, a DNA fragment that codes a 5-50 unit amino acid sequence in that peptide fragment is then prepared, by means of polymerase chain reaction (PCR), synthetic oligonucleotide and the like, linked with a suitable protein and expressed as a fusion protein, and the bondability with the antibody is verified. The protein suitable for use in the fusion protein can be any protein that does not bond with the antibody analyzed. In this way, an epitope which is the smallest polypeptide necessary and sufficient for bonding with the antibody, can be specified.

(B) On the other hand, in the method whereby an antibody, produced by immunization by a specific peptide antigen, is used, a peptide with an amino acid sequence which has already been specified is used as an antigen in the production of the antibody, and the screening of the antibody is also done by using the same peptide. Because the epitope of the antibody, produced in this way, is determined in advance, the peptide can be specified as the epitope of said antibody.

As an example of the method of producing an antibody that specifically recognizes a specific amino acid sequence, contained in a protein, the method described in "Antibodies: A Laboratory Manual", (1989) (Cold Spring Harbor Laboratory Press) can be used. The following is a description of this method in concrete terms.
In order to produce an antibody, an immunogen is first needed. "Immunogen" is a substance that causes an immunoreaction or has the ability to induce one in a normal body. Immunogens can be manufactured using commonly-known methods or methods based on them ("Antibodies: A Laboratory Manual", (1989) (Cold Spring Harbor Laboratory Press)) . Immunogens can be used, as needed, as a complex with a carrier protein such as bovine serum albumin (BSA), bovine thyroglobulin (BTG) and Keyhole Limpet Hemocyanin (KLH) . Since the molecular weight of regular peptides is low, they have no immune responsiveness, which is why it is better to use complexes as immunogens.

Immunity is achieved by administering an immunogen, such as those described above, to a mammal by intravenous, intracutaneous, subcutaneous or intraperitoneal injection. In more specific terms, the immunogen is diluted to the adequate concentration using Phosphate Buffered Saline (PBS), physiological saline solution, or the like, and administered 3 to 10 times at 2 to 6-week gaps to the tested animal, together with a standard adjuvant if needed. Rabbits, goats, sheep, mice, rats and the like are commonly used as the immunized mammal. When mice are used, a single dose is around 50 µg per animal. In this case, when the above-mentioned adjuvant is administered together with the antigen, the adjuvant should be a substance that nonspecifically amplifies the immune reaction against the antigen. Examples of commonly-used adjuvants include pertussis vaccine and Freund's adjuvant. Polyclonal antibodies can be obtained by drawing blood from the mammals 3 to 10 days after the last immunization.

As the method of manufacturing monoclonal antibodies, fused cells (hybridomas) of plasma cells (immunocytes) and tumor cells of plasma cells (myeloma cells) of the mammal immunized by the immunogen are produced, clones that produce monoclonal antibodies that recognize the target antigen are selected out of them, and these clones are cultured. Such monoclonal antibodies can be manufactured basically by implementing a standard method (see Kohler, G. and Milstein, C., Nature, 256, 495-497 (1975)).

In implementing this method, the immunized mammal should be selected taking into consideration the compatibility with the plasma cell tumor cells used for the cell fusion, and mice, rats, or the like can be used.

Hybridomas can be obtained from the immunized cells thus obtained using, for example, the method described in "Experimental Manual for Molecular Cell Biology" (Nankodo Publications, Takekazu Horie et al., 1994), whereby, in order to obtain subculture-able cells, plasma cell tumor cells and antibody-producting immunocytes are fused together in the presence of, for example, polyethylene glycol, yielding hybridomas. As the plasma cell tumor cells used it is recommended to use plasma cell tumor cells from the same homeothermal animal or from congener homeothermal animals; for example, if the fusion is with spleen cells obtained from immunized mice, mouse myeloma cells should be used. As plasma cell tumor cells, commonly-known cells such as p3x63-Ag8.UI can be used.

Hybridomas can be selected by culturing in a standard HAT culture medium (culture medium with added hypoxanthine, aminopterin and tymidine). At the stage that colonies are verified, the hybridomas producing the target antibody can be obtained by checking the bonding of the antibodies discharged in the culture supernatant and the antigen (screening). Examples of screening methods include the spot method, agglutination method, western blot, ELISA and various other methods generally used in the detection of antibodies, but preferably the ELISA method should be used, with the reactivity with the antigen of the supernatant of the hybridoma culture medium serving as an indicator. By means of this screening, the target antibody-producing strain, that specifically reacts with the antigen, can be selected.

Cloning of the target antibody-producing strain can usually be done using the limiting dilution method or the like. If needed, the cloned hybridoma can be mass cultured in a serum-containing medium or in a serum-free medium. By means of such culturing, the target antibody can be obtained at a relatively high level of purity as the culture supernatant. Moreover, the hybridomas can be seeded in the peritoneal cavity of mammals with compatibility with the hybridoma, such as mice, and the target antibody collected in large quantities as the peritoneal fluid of the mice.

The culture supernatant and peritoneal fluid of mice and the like, of the antibody-producing hybridoma pertaining to the present invention, can be used without modification as a crude antibody solution. Furthermore, it can be purified using ammonium sulfate precipitation, salting-out, gel filtration, ion-exchange chromatography, affinity chromatography and the like.

As the epitopes used in the implementation examples of the present invention, polypeptides that contain the amino acid sequences shown in the sequence table as sequence no. 1 and sequence no. 2, derived from type 2 human collagen, which has already been specified by Downs et al. (Journal of Immunological Methods 247, 2001), were used. Moreover, the monoclonal antibodies that specifically recognize these epitopes were produced using the method described in the specification of the present invention.

Specific examples of antibodies that can be used in the method pertaining to the present invention, prepared in the above manner, include the monoclonal antibodies produced by hybridoma TAG-6G4, which are antibodies that specifically bond with a polypeptide containing the amino acid sequence of sequence no. 1 (hereinafter, "6G4 antibodies"), and the monoclonal antibodies produced by hybridoma TAG-2E6, which are antibodies that specifically bond with a polypeptide containing the amino acid sequence of sequence no. 2 (hereinafter, (2E6 antibodies). These hybridomas were deposited, on January 13, 2006, based on the Budapest Treaty concerning international approvals for deposit of patent procedural microorganisms, in the Patent Organism Depositary Center of the Independent Industrial Technology General Laboratories (Chuo 6, Higashi 1-1-1, Tsukuba, Ibaraki Prefecture), as "Mouse-Mouse Hybridoma TAG-2E6" (Receipt No. FERM ABP-10482) and "Mouse-Mouse Hybridoma TAG-6G4" (Receipt No. FERM ABP-10483).

Their antibodies posses the following physicochemical and immunological characteristics. 6G4 antibody
(a) Specifically bonds with peptides consisting of the amino acid sequence GEPGDDAPS.
(b) It is a 150 kDa protein, consisting of an H chain of 50 kDa and an L chain of 27 kDa, which contain a variable region of the H chain (VH region) shown in sequence no. 4 and a variable region of the L chain (VL region) shown in sequence no. 5.
(c) Belongs to immunoglobulin class IgGI(k). 2E6 antibody
(d) Specifically bonds with proteins consisting of the amino acid sequence GPPGPQG.
(e) It is a 150 kDa protein, consisting of an H chain of 50 kDa and an L chain of 27 kDa, which contain a VH region shown in sequence no. 6 and a VL region shown in sequence no. 7.
(f) Belongs to immunoglobulin class IgG2b(k).

The epitope tags can also be used for separation and refinement of the target proteins, using commonly-known techniques. For example, after the quantity of the target protein is measured, using the present invention, the target protein can be separated and refined using the epitope tag used in the present invention and antibodies or the like that recognize that tag. Typically, first a commonly-used column tube or the like is filled with a ligand that is bondable with the epitope tag (such as an antibody, a metal or the like), covalently or non-covalently bonded to a solid surface. Next, a suspension containing the target protein is added to the column and the column is washed with a suitable buffer solution or the like. After washing, the target protein can be eluted and separated from other proteins and components of the culture medium by adding a suitable eluate to the column or by means of a synthetic epitope peptide chain.

The epitope tag used in the method pertaining to the present invention is characterized in that it contains at least two epitopes, placed so that if detection antibodies, that recognize each of the epitopes, are bonded, both said detection antibodies can be brought close to each other. In other words, it means that their position is such that it allows a detection antibody that can specifically bond, via an immune reaction, to one of the epitopes (hereinafter, "epitope 1"), and a detection antibody that can specifically bond, via an immune reaction, to the other epitope (hereinafter, "epitope 2"), to both bond without a steric hindrance, and that when each detection antibody is bonded, a reaction is induced and can be detected. The two epitopes can be directly linked or they may be bridged through a suitable peptide linker. Generally, if the molecular weight of the antibody is 150 KDa, it is bigger than the size of the epitope tag, and each detection antibody prevents bonding of the antibody itself to the epitope by steric hindrance. It is therefore recommended to insert a peptide linker with a suitable length between epitope 1 and epitope 2. In the case that the two epitopes are bridged via a peptide linker, it is recommended to insert between 0 and 20 amino acid residues, preferably between 3 and 6 amino acid residues. Furthermore, although no special restrictions are placed on the amino acid residues used as a linker, they should have no cross-reactivity with the epitope sequences and have relatively small side-chains. Moreover, the amino acid sequences of epitope 1 and epitope 2 do not have to originate from the same species, and can be derived from different species.

Although no special restrictions are placed on the length of the epitope tag, its size should be such that it does not have an effect on the structure of the target protein when it is produced with the target protein as a fusion protein. Usually it should be made of 12 to 40 amino acid residues, preferably between 15 and 22 amino acid residues. Furthermore, in order to prevent polymerization due to cystine formation, the amino acid sequence of the epitope tag should not include cysteine residues. The epitope tag should not only not have biological activity itself, it should also have no effect on the biological activity of the target protein.

A specific example of the epitope tag that can be used in the implementation of the method, pertaining to the present invention, is a polypeptide consisting of a 19-residue amino acid sequence (sequence no. 3), consisting of the amino acid sequence shown in the sequence table as sequence no. 1, and the amino acid sequence shown in the sequence table as sequence no. 2, bonded via a 3-residue peptide linker.

An example of the method of expressing the fusion protein of a target protein, such as described above, and the epitope tag, consisting of epitope 1 and epitope 2, is that whereby the DNA fragments that code the target protein and the epitope tag are bonded so that they match the frame, a suitable expression vector is introduced so that it is under the control of a suitable promoter, and they are transcribed, translated and expressed by a suitable host. No special restrictions are placed on the sequence of bonding the above-mentioned epitope tag and target protein, and they can be bonded to either an amino terminal or a carboxyl terminal. Moreover, insertion of specific amino acid sequences, that proteases (such as enterokinase, factor Xa or the like) recognize, between the epitope tag and target protein, makes it possible to cut out the epitope tag by these proteases.

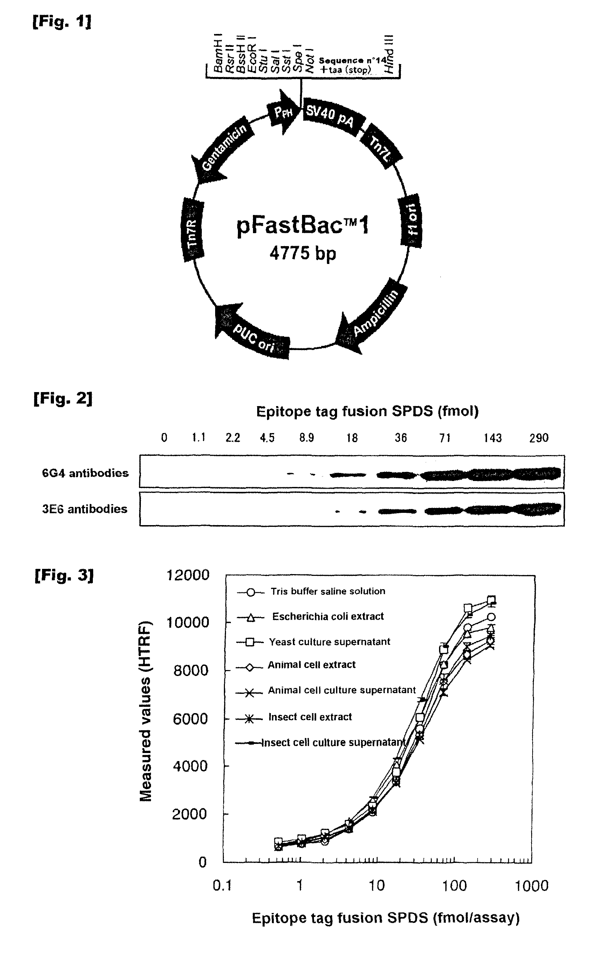

In this process, the preparation of the inserted vectors and the restriction enzyme sites for inserting the gene of the epitope tag and the gene of the target protein is non-specific, and the construction of the expression vector is simple. An example of such an epitope tag fusion protein expression vector is an expression vector with a structure in which sequence no. 14 was inserted as the epitope tag gene, as shown in Fig. 1. Moreover, an expression vector with a gene, consisting of the gene sequence that codes such an epitope tag, can be used as a kit for quantitative measurement of the target protein, by combining it with two or more types of antibodies (for example, 6G4 antibody and 2E6 antibody), that can recognize the epitope tag of the fusion protein expressed, and with detection antibodies, labeled in a manner to be described below. Furthermore, the above kit can be used also in preparing recombinant proteins.

A suitable expression vector can be any vector that suits the host into which it was introduced. Examples of the host include *Escherichia coli,* yeast, animal cells, insect cells etc., and pET, pNMT, pcDNA and pFastBac (all of which can be purchased from Invitrogen), respectively, can be used with these. Moreover, a non-cellular transcription-translation system, prepared based on rabbit reticulocytes, extracted wheat germ, *Escherichia coli* extract *(Escherichia coli* 830 extract) or the like, can also be used as the system for transcription and translation.

The method pertaining to the present invention is characterized in that two types of detection antibodies are brought into contact with an epitope tag fusion protein, expressed in the manner described above. Methods of measuring the quantity of target recombinant proteins, using antibodies capable of bonding immunospecifically to two types of epitopes, are already known (for example, Japanese Patent 14-191364). Nevertheless, when the quantity of a recombinant protein is measured by means of any of these methods, western blot, ELISA or the like, commonly known among those skilled in the art, have been used as a general technique. In the implementation of these methods, separation processes were necessary, such as separating the mixture by means of electrophoresis, or bonding the mixture to a solid surface by using a trapping antibody and then repeatedly washing. For these reasons, the quantitative measurement processes of proteins were complex and required considerable time. By implementing the method pertaining to the present invention, on the other hand, by combining an epitope tag and two types of detection antibodies, the quantity of protein can be rapidly and simply measured, without requiring such a process of separation from other proteins. It can be said to be a useful technique in the production process of recombinant proteins.

With regards to the detection antibodies used in the process, in order to detect the proximity of the antibodies, antibodies labeled with a labeling substance are used. Such detection antibodies may be cultured by addition to a culture medium in which the host is already cultured, by addition after culturing was completed, or by addition after a refining process. The antibodies may be monoclonal or polyclonal, as long as they specifically recognize the polypeptide with the amino acid sequence containing said epitopes, but usage of the former is recommended. The antibodies can be manufactured in accordance with the method described above. Moreover, in order to label the antibodies with a labeling substance, standard methods, such as those described in "Experimental Manual for Molecular Cell Biology" (Nankodo Publications, Takekazu Horie et al., 1994), can be applied, or a manual for labeling substances can be used.

Examples of labeling substances include illuminants, enzymes, fluorescent materials, beads, radioisotopes, metals, biotin and the like. Examples of illuminants include luciphenol, luminol, aequorin, acridinium esther and other chemical illuminants. Examples of enzymes include luciferase, β-galactosidase, alkaline phosphatase, peroxidase and the like. Examples of fluorescent materials include lanthanide elements such as europium (Eu) and terbium (Tb), lanthanide element derivatives such as europium cryptate, fluorescein derivatives such as FITC (fluorescein isothiocyanate), rhodamin derivatives such as RITC (tetramethylrhodamin isothiocyanate), and fluorescent proteins such as YFP, GFP, CFP, BFP allophicocyanine and the like. Examples of beads include protein A beads, wheat germ agglutinin (WGA) beads, streptoavidin beads and other beads that have been subjected to specific treatments. Examples of radioisotopes include ¹⁴C, ¹²⁵I, ³H, ³⁵S and the like. Furthermore, compounds labeled with the above can also be used. Examples of metals include ferritin, gold colloid and the like.

However, when selecting a labeling substance, as mentioned above, a combination that produces a detectable phenomenon when the detection antibodies come close together has to be selected. Generally-known examples of such combinations include a fluorescent material label and a fluorescent material label, an illuminant label and a fluorescent material label, a radioisotope label and beads, and the like.

The method pertaining to the present invention is characterized in that, after two types of detection antibodies are brought into contact in the epitope tag fusion protein, a phenomenon, produced by the coming close together of the two antibodies, is detected. "A phenomenon, produced by the coming close together of the two antibodies, is detected" means that when a detection antibody that recognizes epitope 1 of the above-mentioned epitope tag fusion protein, and a detection antibody that recognizes epitope 2 of the same, bond and form a complex, a phenomenon, produced by the coming close together of the labels of both detection antibodies, is detected. Methods of detecting the interaction between two proteins (detection antibodies) that came close together in such a manner are well known to researchers in this field, and examples thereof include the BRET method, FRET method, AlphaScreen method (registered trademark), SPA method (Scintillation Proximity Assay, registered trademark) and the like.

An example of a favorable method for detecting a phenomenon produced by such a proximity is the method whereby transfer of excitation energy, caused by resonance, is detected. For example, if a fluorescent material is caused to emit excitation light, in an excited state, its energy is released as fluorescence or heat energy, and then returns to the normal state (quenched). If, in that state, another fluorescent material is present close by, a phenomenon is produced whereby that fluorescent material receives the energy release, is further excited, and similarly emits fluorescence. This phenomenon is known as fluorescence resonance energy transfer (hereinafter, FRET). By measuring the fluorescence intensity in that state, using a measuring device such as a luminometer, the target protein present in the solution can be measured.

In such a case it is especially recommended to select a fluorescent material with a long fluorescence lifetime (time period until quenching) as the donor (the molecule that provides energy). By using this method, because the fluorescence of interfering substances such as plastic has a short lifetime, if the fluorescence is measured at set time intervals after the excitation, the effect of background fluorescence can be eliminated. This kind of FRET is called "time-resolved fluorescence resonance energy transfer (hereinafter, TR-FRET). Examples of methods of TR-FRET detection include HTRF (Homogeneous Time-Resolved Fluorescence, registered trademark, CIS Biolnternational) and LANCE (Perkin-Elmer Life Science, registered trademark).

HTRF is characterized in that it used two types of fluorescent materials. Specifically, europium compounds include a certain europium cryptate (hereinafter, "cryptate", a compound with a structure in which europium ions of rare earth elements are arranged in the cage-like structure of trisbipyridine) and the allophycocyanin derivative XL665 (a compound stabilized by 3-molecule crosslinking of allophycocyanin which is a fluorescent protein derived from blue-green algae). If cryptate is irradiated with excitation light at 337 nm, it emits fluorescence with a long lifetime at 620 nm, but if detection antibodies are brought close together and XL665 is present in proximity, because of FRET, the excitation energy is transferred to XL665 and XL665 emits fluorescence with a long lifetime at 665 nm. By measuring this fluorescence with a long lifetime the epitope tag fusion protein can be measured. The advantage of this method is that by measuring the fluorescence at a set time interval after the cryptate excitation, the background fluorescence with a short lifetime (about 10 nanoseconds), caused by fluorescent substances contained in the measured sample and in the measuring tube, is eliminated, and only the fluorescence with a long lifetime (about 1 millisecond) can be selectively detected. Furthermore, by simultaneously measuring the 665 nm fluorescence of XL665 and the 620 nm fluorescence of cryptate, by expressing the measured value as (the fluorescence intensity of 665 nm / the fluorescence intensity of 620 nm) x 10,000, fluctuation in the measured value, caused by differences in the quantity of added reagent and color quenching of the measured sample (the inner filter effect) can also be corrected.

Another mode is called BRET (Bioluminescence resonance energy transfer), and it is a detection method whereby energy transfer due to bioluminescence resonance is used. For example, antibodies labeled with luciferase and with a Green Fluorescent protein mutant (GFP mutant) are used as two types of detection antibodies. When these detection antibodies come close together, part of the energy generated by a luminous reaction between luciferin and luciferase is transferred to the GFP mutant, producing the phenomenon of fluorescence emission (BRET). By measuring the fluorescence intensity at this point, in addition to the quantity of the epitope tag fusion protein, the quantity of the target protein can also be measured.

Other forms include methods whereby a combination of detection antibodies labeled with beads (such as SPA beads, registered trademark) and radioisotopes (such as ³H, ¹⁴C and ¹²⁵I). When two detection antibodies come close together, radioactive rays, such as β-rays, that are emitted by the radioisotope, reach the scintillator inside the beads, and because a fluorescence phenomenon is induced, by detecting the fluorescence, the quantities of the epitope tag fusion protein and of the target protein can be measured.

Moreover, by using an epitope tag fusion protein with a known concentration (hereinafter, "standard protein") in the above-mentioned detection methods, accurate quantitative measurements can be conducted of the epitope tag fusion protein or target protein contained in the tested samples.

Furthermore, the above method, pertaining to the present invention, can be easily executed by using a special kit, and the present invention proposes also such a kit.

The kit pertaining to the present invention contains, as constituents, at least an expression vector and detection antibodies. The expression vector consists of a gene sequence that codes an epitope tag containing epitope 1 and epitope 2, and insertion of the target genes into the vector can be done in accordance with standard methods, by cloning a protein containing at its terminal a tag with two types of epitopes. In addition, the detection antibodies can be of two types or more, and each detection antibody can bond to part of the epitope tag. Moreover, the detection antibodies may be labeled in advance. Furthermore, a host strain capable of highly effective transformation can also be used as a constituent.

The present invention will now be described in specific terms by means of implementation examples. The present invention is not limited, however, to or by these examples. Implementation Example 1

Manufacturing a monoclonal antibody that recognizes an epitope

### (1) Manufacturing 6G4 antibodies

A polypeptide consisting of an amino acid sequence in which cysteine was added to the C-terminal of a region corresponding to amino acid nos. 757 to 765 of a human type 2 collagen (sequence no. 1) and Keyhole Limpet Hemocyanin (KLH, manufactured by Pierce), are conjugated using sulfosuccinimidizole
4-(N-maleimidemethyl)cyclohexane-1-carboxylate (sulfo-SMCC, manufactured by Pierce), yielding an immunogen. The immunogen was mixed with Freund's complete adjuvant (manufactured by Difco) and 40 µg of the emulsion thus produced were intraperitoneally administered every 3 weeks to mice (Balb/c CrSlc, 6 weeks old, female). After the 4^{th} immunization, the mice's spleens were extracted, fused with myeloma cells using the PEG method (p3 x 63-Ag8.UI, Tokyo Cancer Laboratories) and hybridomas were produced. On the 9^{th} day of culturing the culture supernatant was collected and screening was performed for positive wells that contained antibody-producing hybridomas. The screening was done using the time-resolved fluorescence immunoassay (DELFIA, Amersham, registered trademark) described below. 50 µl measurement buffer solution (50 mM tris hydrochloric acid buffer solution (pH 7.5) containing 150 mM NaCl, 0.01% Tween80, 0.5% BSA and 0.05% NaN₃), 50 µl culture supernatant and 50 µl labeled antigen (measurement buffer solution containing 20 ng/ml biotin-labeled target antigen peptide and 100 ng/ml europium-labeled streptoavidine (manufactured by Perkin-Elmer Life Science)) were consecutively added to microtiter plates (manufactured by Sumitomo Bakelite) with solidified anti-mouse IgG antibodies (manufactured by Shibayagi), and a reaction was conducted at 4°C for 16 hours. After washing the plates twice using 200 µl washing solution (150 mM NaCl, 0.02% NaN₃ and 0.01% Tween20), 150 µl enhancement solution (manufactured by Perkin-Elmer Life Science) were added, and the time-resolved fluorescence was measured using 1420ARVOsx Multilabel Counter (manufactured by Perkin-Elmer Life Science). One well with high reactivity was selected by means of this screening, cloning was repeated twice using the limiting dilution method, and the presence of antibody-producing hybridoma TAG-6G4 was established. The TAG-6G4 subclass was determined using a mouse monoclonal antibody isotyping ELISA kit (manufactured by BD BioScience) and was found to be IgG1(k). The hybridomas TAG-6G4 were intraperitoneally administered to nude mice (BALB / cANNCrj-nu-nu) and the peritoneal fluid was collected (entrusted with LaboProducts). Pure antibodies were obtained from the peritoneal fluid by means of affinity chromatography using a protein A column.

### (2) Manufacturing 2E6 antibodies

A polypeptide consisting of an amino acid sequence in which cysteine was added to the N-terminal of a region corresponding to amino acid nos. 769 to 775 of a human type 2 collagen (sequence no. 2) and bovine serum albumin (BSA, manufactured by Pierce) were conjugated using (N-e-maleimide caproyloxy) sulfosuccinimide ester (Sulfo-EMCS, manfuactured by Pierce), yielding an immunogen. In the same manner as with TAG-6G4, the immunogen was administered to mice (A/J Jms Slc, 6 week-old females), their spleens were extracted and cell fusion was conducted. Screening by means of DELFIA and cloning were conducted and the presence of antibody-manufacturing hybridoma TAG-2E6 was established. The antibody subclass was IgG2b(k). Peritoneal fluid was obtained from nude mice and refined antibodies were obtained by means of affinity chromatography using a protein A column.

### (3) Determination of the amino acid sequences of the VH region and VL region

Using RneasyMini kit (manufactured by Qiagen), total RNA was refined from 1 x 10⁷ hybridomas. Using SMART^{™}RACE cDNA amplification kit (manufactured by BD BioScience), the gene sequences of the VH region and VL region were determined by following the operating procedure of the kit. Using SMARTIIA oligonucleotides and a primer specific to the VH region of TAG-6G4 (sequence no. 8), a primer specific to the VH region of TAG-2E6 (sequence no. 9) and a primer specific to the VL region (sequence no. 10), cDNA was synthesized. Using these cDNAs as templates, PCR was conducted using a universal primer and a primer specific to the VH region of TAG-6G4 (sequence no. 11), a primer specific to the VH region (sequence no. 12) and a primer specific to the VL region (sequence no. 13) of TAG-2E6, and the VH gene and VL gene were amplified. The PCR products were cloned using the TOPOTA cloning kit (manufactured by Invitrogen) and their sequences were analyzed (entrusted with Obelon Biotechnology). From the genes thus obtained, the amino acid sequences of the VH region and VL region were determined.

### Implementation Example 2

### Manufacturing of the epitope tag fusion protein

### (1) Manufacturing of the expression vector

The expression vector was manufactured using Gateway technology (Invitrogen). As the target protein, the human spermidine synthetase shown in the sequence table as sequence no. 15 (SPDS, GenBank Accession No. NP003123) was used. After cloning this gene, using methods commonly known to those skilled in the art, an entry vector pENTR11 was inserted via restriction enzyme sites SacI and NotI, yielding an entry clone. Moreover, a gene in which 3 x FLAG tag (manufactured by Sigma) and Reading Frame Cassette A of the Gateway Vector Conversion System were linked, was amplified, a pShuttle vector (manufactured by Clontech) was inserted via the restriction enzyme sites of NheI and KpnI, yielding a destination vector. The entry clone and destination vector were mixed in the presence of Clonase, and by introducing an att intersite site-specific recombination, an expression vector of human SPDS in which a 3 x FLAG tag was connected to the amino terminal was cloned. Furthermore, the ApaI site and NotI site intersite region of the SPDS gene was removed and DNA with a gene sequence (sequence no. 14) that codes an epitope tag was linked to the same region, yielding an expression vector of the epitope tag fusion protein.

### (2) Expression and refinement of the epitope tag fusion protein

Using Lipofectamine 2000 (manufactured by Invitrogen) and the above-mentioned expression vector, and following the procedures manual, HEK293 cells (derived from human fetal kidneys, purchased from American Type Culture Collection) were transfected. After culturing these cells for 2 days they were centrifuged and collected, suspended in ice-stored PBS containing small quantity of protease inhibitor cocktail, and crushed using ultrasound treatment. The crushed cell solution was passed through a carrier column (prepared using Aminolink Solidification Kit, manufactured by Pierce, and following the procedures manual) to which 2E6 antibodies, balanced with PBS, were linked, and thoroughly washed with PBS. Finally, 0.2 M glycine buffer solution (pH 3.0) was passed through the column and the protein elution fraction was collected, neutralized using tris-hydrochloric acid (pH 9.0) and dialyzed using PBS. The protein concentration was determined using protein assay reagents (manufactured by Biorad) and used as a reference material.

### Implementation Example 3

Quantitative measurement of the epitope tag fusion protein using western blot
Western blot was conducted using a general technique (for example, Molecular Cloning: A Laboratory Manual, 3rd Edition (2001)) . Using an assay buffer (50 mM tris-buffer solution (pH 7.4) containing 0.5% BSA and 150 mM NaCl), a 2-fold dilution system of the reference material was prepared and 10 µl of each were developed using 5-10% acrylamide gel (manufactured by Atto) and transcribed to a PVDF membrane (manufactured by Millipore). After the membrane was blocked using Block Ace (manufactured by Dainippon Pharmaceuticals), the 6G4 antibodies or 2E6 antibodies were soaked in Block Ace containing 0.5 to 1 µg/ml and allowed to react for 1 hour at room temperature. After thoroughly washing the membrane with a washing buffer (PBS containing 0.05% Tween20), it was soaked in a solution consisting of HRP-labeled anti-mouse IgG antibodies (manufactured by Amersham) 5,000 times diluted in Block Ace, and a reaction was conducted at room temperature for 0.5 hours. The membrane was washed again, and following the procedures manual, was caused to react with a chemiluminescent reagent ECL plus (manufactured by Amersham), and exposed to HyperFilm-ECL (manufactured by Amersham).

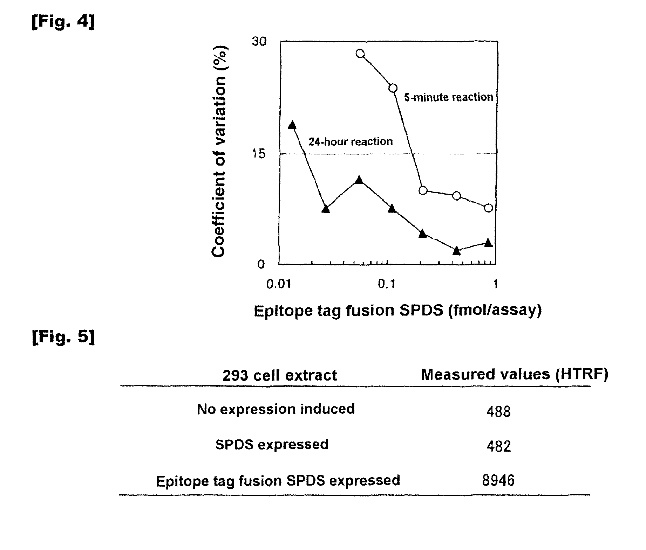

(Results) The measurement results of the epitope tag fusion protein, by means of western blot, are shown in Fig. 2.
When either 6G4 antibodies or 2E6 antibodies were used, single bands, thought to correspond to the epitope tag fusion protein, were obtained at the molecular weight 3.5 kDa location. These bands were not strongly dependent on the quantity of the reference material used. Moreover, the detection limit of the epitope tag fusion protein was 8.9 fmol when 6G4 antibodies were used and 18 fmol when 2E6 antibodies were used.

### Implementation Example 4

Quantitative measurement of epitope fusion proteins by means of the method pertaining to the present invention

### (1) Cryptate labeling 6G4 antibodies

The 6G4 antibody's concentration was adjusted to 1 mg/ml using 0.1 M phosphoric acid buffer solution (pH 8.0). 15 mol cryptate TBP monosuberate (manufactured by CIS BioInternational) were added to 1 mol antibodies and a reaction was conducted for 1 hour at 25°C. The reaction solution was subjected to gel filtration, using a PD-10 column (manufactured by Amersham) that was balanced in advance with PBS, and labeled antibodies were isolated. 0.1% bovine serum albumin, 0.1% Tween20 and 0.05% sodium azide were added to the labeled antibodies and they were kept at -70°C.

### (2) XL665 labeling of 2E6 antibodies

2E6 antibodies, dissolved in 0.1 M phosphoric acid buffer solution (pH 7.0), were used, 8 times the molar quantity of N-succinimidyl 3-(2-pyridil dithio)propionate (SPDP, manufactured by Pierce) were added and a reaction was conducted for 20 minutes at 25°C. Dithiothreitol (DTT) at a final concentration of 10 mMwas added to the reaction solution and a reaction was further conducted for 10 minutes, gel filtration was conducted using a PD-10 column that was balanced using a 0.1 M phosphoric acid buffer solution (pH 7.0) containing 10 mM EDTA, and 2E6 antibodies to which a cysteine group was introduced were isolated (SH-2E6 antibodies) . XL665 (manufactured by CIS Biolnternational) was diluted with 0.1 M phosphoric acid buffer solution (pH 7.0), 5 times the molar quantity of Sulfo-SMCC were added, and a reaction was conducted for 30 minutes at 25°C. Gel filtration was conducted using a PD-10 column that was balanced in advance using a 0.1 M phosphoric acid buffer solution (pH 7.0) and XL665 to which a maleimide group was introduced (maleimide XL665) was isolated. 3 mols of the above-mentioned SH-2E6 antibodies were added to 1 mol maleimide XL665 and a reaction was conducted for 16 hours at 4°C, yielding XL665-labeled antibodies. 100 times the molar quantity of N-ethylmaleimide were added to the labeled antibodies, the mixture was left alone for 10 minutes at room temperature, 0.1% bovine serum albumin, 0.1% Tween20 and 0.05% sodium azide were added and they were kept at -70°C.

### (3) Measurements of the epitope tag fusion protein

Assay buffer (tris buffer saline solution containing 0.5% BSA (TBS)) and extracts of crushed animal cells (HEK 293 cells), insect cells (Sf9 cells), fission yeast and *Escherichia coli,* or culture supernatants thereof, were used, and 2-fold dilution systems of reference materials (see Implementation Example 2) were prepared. 10 µl of each of the reference material diluted solutions thus obtained, were put into 1 well of a 384-well assay plate, 10 µl assay buffer (TBS containing 200 ng/ml cryptate-labeled 6G4 antibodies, 5,000 ng/ml XL665-labeled 2E6 antibodies, 0.8 M potassium fluoride and 0.5% BSA) containing two types of detection antibodies were added and a reaction was conducted at room temperature. The fluorescence intensity of each well was measured 5 minutes and 24 hours after the beginning of the reaction, using Rubystar (manufactured by BMG Labtechnologis).
Next, using the plasmid for expression, manufactured in the manner described in Implementation Example 2, and by means of lipofectamine 2000 (manufactured by Invitrogen), HEK293 cells were transfected and cultured for 3 days at 37°C, in the presence of 5% CO₂, the cells were collected by means of centrifugation and suspended in PBS containing a protease inhibitor cocktail (manufactured by Roche). The cells were crushed by ultrasound and the proteins present in the extract were measured using the method pertaining to the present invention.

(Results) Fig. 3 shows calibration curves of the epitope tag fusion protein (SPDS) in extracts of the animal cells, *Escherichia coli,* fission yeast and insect cells, and the culture medium component. The results show almost no effect of the samples on the measurement method pertaining to the present invention. The results suggest, therefore, that the quantitative measurement method pertaining to the present invention can be directly used on samples of various expression systems without refining them.
Next, the coefficients of variation of the measured value (CV, the ratio of standard deviation with respect to the mean) were calculated (n = 16) for the reference materials with known concentrations prepared with buffers used in the assay. When the coefficient of variation shows "under 15%", the protein concentration shown is the determination limit. Fig. 4 shows the relationships between the reference material concentration and the coefficient of variation (%).
When measurements were conducted using the method pertaining to the present invention, the determination limit after 5 minutes was 2.1 fmol (210 pmol/L). After 24 hours, the determination limit was 0.27 fmol (27 pmol/L). Either value was higher than the detection limit in western blot (8.9 fmol, 18 fmol). These results indicate that, in determining the expressed quantity of a target epitope tag fusion protein, while the western blot requires about 2 days until the results are confirmed, the method pertaining to the present invention enables measurement with the same precision within only 5 minutes. Its precision exceeded that of western blot also after 24 hours, and it can be used also for measuring recombinant proteins, which can be expressed only in small quantities.
Moreover, in practice, the results obtained when a target protein (SPDS) was expressed by HEK293 cells, and the expressed quantity of the target protein (SPDS) contained in the extract was measured, using the method pertaining to the present invention, showed that the measured value of the epitope tag fusion protein (SPDS) contained in the extract could be obtained after merely a 5-minute reaction (Fig. 5). Based on the above results it can be said that the quantitative measurement method for epitope tag fusion proteins, pertaining to the present invention, is superior to the existing methods, such as western blot, in that it is rapid and simple, and in that it can measure quantities with high sensitivity.

Sequence no. 1 is the polypeptide sequence used to manufacture antibodies.
Sequence no. 2 is the polypeptide sequence used to manufacture antibodies.
Sequence no. 3 is the polypeptide sequence used as an epitope tag.
Sequence no. 4 is the amino acid sequence of the variable region (VH) of the 6G4 antibody.
Sequence no. 5 is the amino acid sequence of the variable region (VL) of the 6G4 antibody.
Sequence no. 6 is the amino acid sequence of the variable region (VH) of the 2E6 antibody.
Sequence no. 7 is the amino acid sequence of the variable region (VL) of the 2E6 antibody.
Sequence no. 8 is an oligonucleotide designed to synthesize the cDNA of the VH region of the 6G4 antibody.
Sequence no. 9 is an oligonucleotide designed to synthesize the cDNA of the VH region of the 2E6 antibody.
Sequence no. 10 is an oligonucleotide designed to synthesize the cDNA of the VL regions of the 6G4 antibody and 2E6 antibody. Sequence no. 11 is a PCR oligoprimer designed to amplify the gene of the VH region of the 6G4 antibody.
Sequence no. 12 is a PCR oligoprimer designed to amplify the gene of the VH region of the 2E6 antibody.
Sequence no. 13 is a PCR oligoprimer designed to amplify the gene of the VL region of the 6G4 antibody and 2E6 antibody. Sequence no. 14 is the gene sequence that codes the amino acid sequence of the epitope tag mentioned in sequence no. 3.
Sequence no. 15 is the cDNA of human spermidine synthetase (SPDS).

## Claims

1. A method for quantitative measurement of target proteins, which is **characterized in that** it consists of a process in which, in the fusion protein of the epitope tag, containing epitope 1 and epitope 2, and the target protein, the detection antibody that recognizes epitope 1 and the detection antibody that recognizes epitope 2 are brought into contact, and the phenomenon produced by bringing close together said two detection antibodies is detected, and **in that** said epitope 1 and epitope 2, in the case that the detection antibodies that recognize each of them are bonded, are placed so that these two detection antibodies can be brought close together.

2. The method according to Claim 1, whereby said detection antibodies are labeled by means of a fluorescent material, illuminant, radioisotope or beads.

3. The method according to Claim 2, whereby said detection antibodies are labeled by a fluorescent material.

4. The method according to Claim 3, whereby said fluorescent material is a combination of a europium compound and an allophycocyanin derivative.

5. The method according to any of Claims 1 through 4, whereby said phenomenon is energy transfer by fluorescent resonance.

6. The method according to Claim 1, whereby in one of said detection antibodies, the amino acid sequence of the VH region is the one shown in sequence no. 4 and the amino acid sequence of the VH region is the one shown in sequence no. 5.

7. The method according to Claim 1 or 6, whereby in one of said detection antibodies, the amino acid sequence of the VH region is the one shown in sequence no. 6 and the amino acid sequence of the VH region is the one shown in sequence no. 7.

8. The method according to Claim 1, whereby each of said epitopes consists of 6 to 8 amino acid residues.

9. The method according to Claim 8, whereby the amino acid sequence of one of said epitopes is the one shown in sequence no. 1.

10. The method according to Claim 8 or 9, whereby the amino acid sequence of one of said epitopes is the one shown in sequence no. 2.

11. The method according to Claim 1, whereby epitope 1 and epitope 2 are mutually linked via a peptide linker.

12. The method according to Claim 11, whereby said peptide linker consists of 3 to 6 amino acid residues.

13. The method according to Claim 1, whereby said epitope tag consists of 15 to 22 amino acid residues.

14. The method according to Claim 13, whereby the amino acid sequence of said epitope tag is the one shown in sequence no. 3.

15. An expression vector containing a gene consisting of the sequence shown in sequence no. 14.

16. A kit for quantitative measurement of target proteins, containing detection antibodies that recognize epitope 1, detection antibodies that recognize epitope 2, and an expression vector that contains a gene consisting of a gene sequence that codes the epitope tag that consists of epitope 1 and epitope 2.

17. A kit for quantitative measurement of target proteins according to Claim 16, containing the detection antibodies according to Claim 6, the detection antibodies according to Claim 7, and the expression vector according to Claim 15.

18. A kit which uses the method according to Claim [1].

19. A detection antibody that recognizes epitope 1 and a detection antibody that recognizes epitope 2, and a fusion protein complex of an epitope tag, consisting of epitope 1 and epitope 2, and the target protein.


SEQUENCE LISTING

<110>  Shionogi&Co., Ltd.

<120>  A novel method of recombinant protein measurement

<130>  05P00163

<140>  PCT/JP2006/301226
<141>  2006-01-26

<150>  JP2005-020856
<151>  2005-01-28

<160>  15

<170>  PatentIn version 3.1

<210>  1
<211>  9
<212>  PRT
<213>  Artificial

<220>
<223>  polypeptide for antigen

<400>  1

Gly Glu Pro Gly Asp Asp Ala Pro Ser
1               5


<210>  2
<211>  7
<212>  PRT
<213>  Artificial

<220>
<223>  polypeptide for antigen

<220>
<221>  MOD_RES
<222>  (3)..(3)
<223>  4Hyp


<400>  2

Gly Pro Pro Gly Pro Gln Gly
1               5


<210>  3
<211>  19
<212>  PRT
<213>  Artificial

<220>
<223>  polypeptide for epitope tag

<400>  3

Gly Glu Pro Gly Asp Asp Gly Pro Ser Gly Ala Glu Gly Pro Pro Gly
1               5                   10                  15


Pro Gln Gly

<210>  4
<211>  116
<212>  PRT
<213>  Mus musculus

<400>  4

Glu Val Gln Leu Val Glu Ser Gly Gly Ser Leu Val Gln Pro Gly Gly
1               5                   10                  15

Ser Leu Lys Leu Ser Cys Ala Ala Ser Gly Phe Thr Phe Asn Asn Tyr
            20                  25                  30

Gly Met Ser Trp Val Arg Gln Ile Pro Asp Lys Arg Leu Glu Leu Val
        35                  40                  45

Ala Thr Ile Thr Gly Asn Gly His Ser Thr Tyr Tyr Pro Val Ser Val
    50                  55                  60

Arg Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Val Arg Gly Tyr Ser Asn Phe Ala Phe Trp Gly Gln Gly Thr Thr Leu
            100                 105                 110

Thr Val Ser Ser
        115

<210>  5
<211>  113
<212>  PRT
<213>  Mus musculus

<400>  5

Asp Val Val Leu Thr Gln Thr Pro Leu Thr Leu Ser Leu Thr Ile Gly
1               5                   10                  15

Gln Pro Ala Ser Ile Ser Cys Lys Ser Ser Gln Ser Leu Leu Asn Ser
            20                  25                  30

Asp Gly Glu Thr Tyr Leu Asn Trp Leu Leu Gln Arg Pro Gly Gln Ser
        35                  40                  45

Pro Lys Arg Leu Ile Tyr Leu Val Ser Lys Leu Gly Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Cys Gln Gly
                85                  90                  95

Thr His Phe Pro Trp Thr Phe Gly Gly Gly Thr Lys Leu Glu Ile Lys
            100                 105                 110

Arg

<210>  6
<211>  120
<212>  PRT
<213>  Mus musculus

<400>  6

Gln Val Thr Leu Lys Glu Ser Gly Pro Gly Ile Leu Gln Pro Ser Gln
1               5                   10                  15

Thr Leu Ser Leu Thr Cys Ser Phe Ser Gly Phe Ser Leu Ser Thr Tyr
            20                  25                  30

Gly Met Gly Val Gly Trp Ile Arg Gln Pro Ser Gly Lys Gly Leu Glu
        35                  40                  45

Trp Leu Ala His Ile Trp Trp Asn Asp Asn Lys Tyr Tyr Asn Thr Thr
    50                  55                  60

Leu Lys Ser Arg Leu Thr Ile Ser Lys Asp Thr Ser Asn Lys Gln Val
65                  70                  75                  80

Phe Leu Lys Ile Ala Ser Val Asp Thr Ala Asp Thr Ala Thr Tyr Tyr
                85                  90                  95

Cys Ala Arg Ile Glu Arg Trp Asp Pro Trp Phe Ala Tyr Trp Gly Gln
            100                 105                 110

Gly Thr Leu Val Thr Val Ser Ala
        115                 120

<210>  7
<211>  113
<212>  PRT
<213>  Mus musculus

<400>  7

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val Tyr Ser
            20                  25                  30

Asn Gly Asn Thr Tyr Phe His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Leu Thr Phe Gly Ala Gly Thr Lys Leu Glu Leu Lys
            100                 105                 110

Arg

<210>  8
<211>  24
<212>  DNA
<213>  Artificial

<220>
<223>  primer for cDNA synthesis

<400>  8
cagggtcacc atggagttag tttg                                              24

<210>  9
<211>  24
<212>  DNA
<213>  Artificial

<220>
<223>  primer for cDNA synthesis

<400>  9
tccagagttc caagtcacag tcac                                              24

<210>  10
<211>  24
<212>  DNA
<213>  Artificial

<220>
<223>  primer for cDNA synthesis

<400>  10
gactgaggca cctccagatg ttaa                                              24

<210>  11
<211>  30
<212>  DNA
<213>  Artificial

<220>
<223>  primer for PCR

<400>  11
aggggccagt ggatagacag atgggggtgt                                        30

<210> 12
<211> 30
<212> DNA
<213> Artificial

<220>
<223> primer for PCR

<400> 12
aggggccagt ggatagactg atgggggtgt 30


<210> 13
<211> 33
<212> DNA
<213> Artificial

<220>
<223> primer for PCR

<400> 13
ggatggtggg aagatggata cagttggtgc agc 33


<210> 14
<211> 57
<212> DNA
<213> Artificial

<220>
<223> Designed oligonucleotide to synthesize epitope tag

<400> 14
ggagagcctg gagatgacgg tccctctggt gccgaaggtc caccaggtcc ccagggt 57


<210> 15
<211> 909
<212> DNA
<213> Homo sapiens

<400> 15
atggagcccg gccccgacgg ccccgccgcc tccggccccg ccgccatccg cgagggctgg 60

ttccgcgaga cctgcagcct gtggcccggc caggccctgt cgctgcaggt ggagcagctg 120

ctccaccacc ggcgctcgcg ctaccaggac atcctcgtct tccgcagtaa gacctatggc 180

aacgtgctgg tgttggacgg tgtcatccag tgcacggaga gagacgagtt ctcctaccag 240

gagatgatcg ccaacctgcc tctctgcagc caccccaacc cgcgaaaggt gctgatcatc 300

gggggcggag atggaggtgt cctgcgggag gtggtgaagc acccctccgt ggagtccgtg 360

gtccagtgtg agatcgacga ggatgtcatc caagtctcca agaagttcct gccaggcatg 420

gccattggct actctagctc gaagctgacc ctacatgtgg gtgacggttt tgagttcatg 480

aaacagaatc aggatgcctt cgacgtgatc atcactgact cctcagaccc catgggcccc 540

gccgaaagtc tcttcaagga gtcctattac cagctcatga agacagccct caaggaagat 600

ggtgtcctct gctgccaggg cgagtgccag tggctgcacc tggacctcat caaggagatg 660

cggcagttct gccagtccct gttccccgtg gtggcctatg cctactgcac catccccacc 720

taccccagcg gccagatcgg cttcatgctg tgcagcaaga acccgagcac gaacttccag 780
gagccggtgc agccgctgac acagcagcag gtggcgcaga tgcagctgaa gtactacaac 840
tccgacgtgc accgcgccgc ctttgtgctg cccgagtttg cccgcaaggc cctgaatgat 900
gtgagctga 909